**Europäisches Patentamt**

**European Patent Office**

**Office européen des brevets**

(19)

(11) Publication number : **0 408 713 B1**

# (12) EUROPEAN PATENT SPECIFICATION

(45) Date of publication of patent specification :
**19.05.93 Bulletin 93/20**

(51) Int. Cl.⁵ : **C07C 317/36, A61K 31/10**

(21) Application number : **90902305.3**

(22) Date of filing : **02.02.90**

(86) International application number :
**PCT/GB90/00150**

(87) International publication number :
**WO 90/08761 09.08.90 Gazette 90/19**

(54) **PHENYLSULPHONE DERIVATIVES.**

(30) Priority : **06.02.89 US 306676**

(43) Date of publication of application :
**23.01.91 Bulletin 91/04**

(45) Publication of the grant of the patent :
**19.05.93 Bulletin 93/20**

(84) Designated Contracting States :
**AT BE CH DE DK ES FR GB IT LI LU NL SE**

(56) References cited :
**EP-A- 0 304 190**

(73) Proprietor : **IMPERIAL CHEMICAL INDUSTRIES PLC**
**Imperial Chemical House, Millbank**
**London SW1P 3JF (GB)**

(72) Inventor : **BRITTAIN, David, Robert**
**23 Springbank Lane Bamford**
**Rochdale Lancashire OL11 5SE (GB)**

Inventor : **BROWN, Steven, Paul Galdwill Bungalow**
**Bowden Lane Stratton**
**Bude Cornwall EX23 9BH (GB)**
Inventor : **COOPER, Anthony, Loren**
**Higher Grimscott Launcells**
**Bude Cornwall EX23 9LU (GB)**
Inventor : **LONGRIDGE, Jethro, Lawrence**
**15 Henbury Rise Henbury**
**Macclesfield Cheshire SK11 9NW (GB)**
Inventor : **MORRIS, Jeffrey James**
**14 Armitstead Road Wheelock**
**Sandbach Cheshire CW11 9LP (GB)**
Inventor : **PRESTON, John**
**5 Ashworth Park Knutsford**
**Cheshire WA16 9DE (GB)**
Inventor : **SLATER, Linda**
**17 Dalesford Crescent Macclesfield**
**Cheshire SK10 3LE (GB)**

(74) Representative : **Smith, Stephen Collyer et al**
**ICI Group Patents Services Dept. PO Box 6**
**Shire Park Bessemer Road**
**Welwyn Garden City Herts, AL7 1HD (GB)**

## Description

This invention concerns novel phenylsulphone derivatives which are inhibitors of the enzyme aldose reductase and which are of value, for example, in the treatment of certain peripheral effects of diabetes or galactosemia. A method of treating one or more of such peripheral effects using a phenylsulphone derivative and pharmaceutical compositions containing such a derivative are also provided. In addition, the invention concerns novel processes for the manufacture of the novel derivatives and for the preparation of medicaments containing any of the said derivatives.

## Background to Invention

The enzyme aldose reductase is responsible for the catalytic conversion of aldoses, such as glucose and galactose, to the corresponding alditols, such as sorbitol and galactitol respectively, in warm blooded animals such as man. Alditols penetrate cell membranes poorly and, once formed, tend to be removed only by further metabolism. Consequently, alditols tend to accumulate within cells where they are formed, causing a rise in internal osmotic pressure which may in turn be sufficient to destroy or impair the function of the cells themselves. In addition, raised alditol levels may result in abnormal levels of their metabolites which may themselves impair or damage cellular function. The enzyme aldose reductase has a relatively low substrate affinity and is generally only effective in the presence of relatively large concentrations of aldose. Such large concentrations are present in the clinical conditions of diabetes (excessive glucose) and galactosemia (excessive galactose). Consequently, aldose reductase inhibitors are useful in the reduction or prevention of the development of those peripheral effects of diabetes or galactosemia which may be due in part to the accumulation of sorbitol or galactitol, respectively, in tissues such as the eye, nerve and kidney. Such peripheral effects include, for example, macular oedema, cataract, retinopathy, neuropathy and impaired neural conduction.

Although a number of aldose reductase inhibitors have been discovered and clinically evaluated, there is a continuing need for alternative inhibitors. In our European patent application, publication number 304,190, there is described a series of (phenylsulphonyl)nitromethane derivatives as inhibitors of the enzyme aldose reductase. We have now discovered that a specific, novel (phenylsulphonyl)nitromethane derivative set out below is an unexpectedly potent inhibitor of aldose reductase and this is a basis for the present invention.

## Disclosure of Invention

According to the invention there is provided the novel compound (4-amino-2,6-dimethylphenylsulphonyl)nitromethane (hereinafter referred to as "the Compound"), or a pharmaceutically acceptable salt thereof.

According to another aspect of the invention there is provided a pharmaceutical composition comprising the Compound, or a pharmaceutically acceptable salt thereof, together with a pharmaceutically acceptable diluent or carrier.

The compositions of the invention may be in various conventional forms. Thus, they may be in a form suitable for oral use (for example as tablets, lozenges, hard or soft capsules, aqueous or oily suspensions, emulsions, dispersible powders or granules, syrups or elixirs), for topical use (for example as creams, ointments, gels or aqueous or oily solutions or suspensions) or for parenteral administration (for example as a sterile aqueous or oily solution for intravenous, subcutaneous, intramuscular or intravascular dosing) or as a suppository for rectal dosing.

The compositions of the invention may be obtained by conventional procedures using conventional pharmaceutical excipients, well known in the art. Thus, compositions intended for oral use may contain, for example, one or more colouring, sweetening, flavouring and/or preservative agents and may be in the form of hard gelatin capsules in which the active ingredient is mixed with an inert solid diluent, for example, calcium carbonate, calcium phosphate or kaolin, Compositions for oral use may also be in the form of soft gelatin capsules in which the active ingredient is mixed with water or an oil such as arachis oil, liquid paraffin or olive oil.

Suitable pharmaceutically acceptable excipients for use in tablet formulations include, for example, inert diluents such as lactose, sodium carbonate, calcium phosphate or calcium carbonate, granulating and disintegrating agents such as corn starch or alginic acid; binding agents such as gelatin or starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Aqueous suspensions will generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxyethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. Aqueous suspensions will also typically contain one or more preservatives (such as ethyl or propyl p-hydroxybenzoate, anti-oxidants (such as ascorbic acid), colouring agents, flavouring agents, and/or sweetening agents (such as sucrose, saccharin or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, or esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Suppository formulations may be prepared by mixing the active ingredient with a suitable non-irritating excipient which is solid at ordinary temperatures but liquid at the rectal temperature and will therefore melt in the rectum to release the drug. Suitable excipients include, for example, cocoa butter and polyethylene glycols.

Topical formulations, such as creams, ointments, gels and aqueous or oily solutions or suspensions, may generally be obtained by formulating an active ingredient with a conventional, topically acceptable, vehicle or diluent using conventional procedures well known in the art. Topical formulations for administration to the eye will generally be in the form of an ointment, gel or sterile solution buffered at an ophthalmically acceptable pH, for example in the range pH 7.0-7.6.

The amount of active ingredient that is combined with one or more excipients to produce a single dosage form will necessarily vary depending upon the host treated and the particular route of administration. For example, a formulation intended for oral administration to humans will generally contain for example from 0.5 mg to 1g of active agent compounded with an appropriate and convenient amount of excipients which may vary from about 5 to about 98 percent by weight of the total composition. Dosage unit forms will generally contain about 1 mg to about 500 mg of an active ingredient.

Particularly suitable salts include, for example, pharmaceutically acceptable base-addition salts, for example, alkali metal (such as potassium or sodium), alkaline earth metal (such as calcium or magnesium) and ammonium salts, and salts with organic bases giving physiologically acceptable cations (such as salts with methylamine, dimethylamine, trimethylamine, piperidine and morpholine); and pharmaceutically acceptable salts with acids (such as salts with hydrogen halides [especially hydrogen chloride and hydrogen bromide), sulphuric acid and phosphoric acid].

The Compound of the invention may be obtained by standard procedures of organic chemistry already known for the production of structurally analogous compounds, for example by one or more of the procedures described in our aforementioned European patent application or as reviewed in the paper by Zeilstra et alia in Rec. Trav. Chim. Pays Bas 1974, 93, 11-14. Such procedures are provided as a further feature of the invention and are illustrated by the following procedures.

(a) Removing a protecting group from a protected form of the Compound.

Suitable protecting groups and the procedures necessary for their introduction and removal are well known and are described in standard text-books of organic chemistry.

A particularly suitable protected form of the Compound is, for example, an N-acyl derivative thereof, and especially an N-(1-6C)alkanoyl or benzoyl derivative, such as the N-acetyl, N-propanoyl, N-benzoyl or N-p-chlorobenzoyl derivative of the Compound.

The protecting group may be removed by conventional procedures. Thus, for example, N-acyl derivatives, may conveniently be removed by hydrolysis, for example by reaction with a strong acid such as hydrochloric, phosphoric or sulphuric acid, or by rection with a strong base, for example an alkali metal hydroxide such as lithium, sodium or potassium hydroxide. In either case, the hydrolysis is generally performed in an aqueous medium (such as water optionally together with a (1-4C)alkanol such as ethanol) and at a temperature in the range, for example, 40 to 100°C.

The starting protected forms of the Compound may conveniently be obtained, for example, by introduction of a protecting group onto the amino group early in the synthetic sequence. Thus, for example, N-alkanoyl or benzoyl derivatives of the Compound may be obtained by reacting 3,5-dimethylaniline with the appropriate alkanoyl (or benzoyl) chloride, bromide or anhydride, to give the corresponding N-acyl-3,5-dimethylaniline. The acylation is generally performed with an excess of the acylating agent in the presence of a base such as triethylamine in a suitable solvent or diluent such as t-butyl methyl ether or tetrahydrofuran and at a temperature of, for example, 10 to 40°C and conveniently at or near ambient temperature. The N-acyl-3,5-dimethylaniline is then chlorosulphonated by reaction with chlorosulphonic acid to give the (4-N-acylamino-2,6-dimethylphenyl)sulphonyl chloride, which latter is reduced, for example, with a suitable sulphite (such as sodium sulphite) in the presence of a suitable buffer (such as sodium hydrogen carbonate) at a temperature of, for example, 60 to 90°C, to give the (4-N-acylamino-2,6-dimethylphenyl)sulphinic acid. The latter acid is then converted to its alkali metal salt and then reacted with nitromethane in the presence of iodine and an alkali metal (1-6C)alkoxide such as potassium t-butoxide to give the required (4-N-acylamino-2,6-dimethylphenylsulphonyl)nitromethane. The latter step is analogous to process (b) described below. The overall procedure is illustrated in the accompanying Examples.

The N-acyl derivatives of the Compound are novel and are provided as a further aspect of the invention. Certain of the N-acyl derivatives, such as the N-acetyl derivative (4-acetamido-2,6-dimethylphenylsulphonyl)nitromethane, also possess the property of inhibiting the enzyme aldose reductase.

(b) Reacting an alkali metal salt of 4-amino-2,6-dimethylbenzene sulphinic acid, with nitromethane and iodine in the presence of an alkali metal (1-6C)alkoxide such as potassium t-butoxide or sodium methoxide.

The reaction is preferably carried out in the presence of a suitable polar solvent, for example, 1,3-dimethyl-3,4,5,6-tetrahydro-2-(1H)-pyrimidinone (DMPU) or N,N-dimethylformamide (which are preferred), or N-methyl-2-pyrrolidone, and at a temperature in the range, for example, -30 to 20°C and, conveniently, at about 0°C. The nitromethane is generally present in an excess.

The starting alkali metal salt may be obtained, for example, from the corresponding sulphinic acid by reaction with the appropriate alkali metal hydroxide or (1-6C)alkoxide, such as sodium or potassium methoxide or ethoxide. The sulphinic acid may itself be obtained in an anogous manner to the corresponding N-acyl derivative mentioned in process (a) above, for example, by a conventional reduction of 4-amino-2,6-dimethylbenzene sulphonyl chloride using, for example, aqueous sodium sulphite and sodium hydrogen carbonate. The sulphonyl chloride may be obtained, for example, from 4-amino-2,6-dimethylphenyl isothiocyanate by reaction with chlorine in water, using conditions analogous to those described by Johnson et alia in J. Amer. Chem. Soc., 1939, 61, 2548. The isothiocyanate may itself be obtained, for example, by reaction of 3,5-dimethylaniline with thiocyanogen (generated in situ from lead(II) thiocyanate and chlorine in acetic acid) or copper(II) thiocyanate in methyl or ethyl acetate.

(c) Oxidising the thioether, (4-amino-2,6-dimethylphenylthio)nitromethane.

Suitable oxidising agents include, for example, those which are well known in the art for the conversion of thio to sulphonyl groups and which are compatible with the amino group. Thus, for example, an alkali metal periodate (such as sodium metaperiodate), persulphate (such as potassium monopersulphate) or permanganate (such as potassium permanganate) may be employed.

The oxidation is preferably carried out in a suitable conventional solvent or diluent for such oxidations, for example in acetic or propionic acid, and at a temperature in the general range, for example 0 to 80°C.

In certain cases, the partial oxidation product (4-amino-2,6-dimethylphenylsulphinyl)nitromethane may be produced as an isolable intermediate. This product may then be further oxidised to give the Compound. The invention also includes the oxidation of such an intermediate to the Compound, for example, by reaction with an alkali metal permanganate (such as potassium permanganate) in a suitable solvent such as aqueous acetic acid and at a temperature in the range, for example, 20 to 80 °C.

The starting thioether may be obtained by conventional procedures of organic chemistry, for example, from the potassium or sodium salt of 4-amino-2,6-dimethylphenylthiol by conversion to the corresponding thioacetic acid (or a (1-4C)alkyl ester thereof, such as a methyl or ethyl ester) by reaction with chloro- or bromoacetic acid (or a (1-4C)alkyl ester thereof) in the presence of a suitable base. The acid (or (1-4C)alkyl ester thereof) is then reacted with a (1-5C)alkyl nitrate and an alkali metal (1-6C)alkane, for example isopropyl nitrate and butyllithium, in a suitable solvent or diluent, such as tetrahydrofuran or t-butyl methyl ether, and at a temperature in the range, for example, -80 to 10°C, to give the alkali metal salt of the 2-(4-amino-2,6-dimethylphenylthio)-2-nitroacetic acid (or an (1-4C)alkyl ester thereof). The latter acid is unstable and readily decarboxylates. Acidification of the alkali metal salt of the nitroacetic acid allows the isolation of the required thioether. Similarly, an alkyl ester of the nitroacetic acid may be hydrolysed, for example, using aqueous base, to the corresponding acid and then acidified to produce the thioether.

4-Amino-2,6-dimethylphenylthiol may be obtained, for example by reaction of 3,5-dimethylaniline with thiocyanogen (generated in situ from lead(II) thiocyanate and chlorine in acetic acid or with copper(II) thiocyanate to give 4-amino-2,6-dimethylphenyl isothiocyanate, which latter is then reduced, for example, with sodium borohydride in ethanol to the required thiol.

Whereafter, when a pharmaceutically acceptable salt is required, the Compound may be reacted with an appropriate base or acid having a physiologically acceptable ion.

As stated previously, the Compound inhibits the enzyme aldose reductase and is thus of value, for example, in treating those diseases or conditions which are caused by excessive quantities of the products such as sorbitol formed in the body by processes catalysed by the enzyme aldose reductase.

The property of inhibiting the enzyme aldose reductase in vivo may be demonstrated in the following standard laboratory test:-

Rats are made diabetic (as evidenced by severe glucosuria being present) by dosing with streptozotocin. The animals are then dosed daily with the test compound for one, two or five days. The animals are then sacrificed 2-6 hours after the final dose and the eye lenses and/or sciatic nerves are removed. After a standard work-up procedure the residual sorbitol levels in each tissue are determined by gas liquid chromatography after conversion to the polytrimethylsilyl derivatives. Inhibition of aldose reductase in vivo can then be assessed by comparing the residual sorbitol levels in tissues from the dosed diabetic group of rats with those of an undosed group of diabetic rats and an undosed, normal group of rats.

In typical examples of the above in vivo test, the Compound had an $ED_{50}$ for oral administration of about 0.9 mg/kg. By contrast, the structurally analogous (4-aminophenylsulphonyl)nitromethane, described in our aforementioned European patent application, had an oral $ED_{50}$ of about 4.8 mg/kg.

The property of inhibiting the enzyme aldose reductase may also be demonstrated in vitro. Thus, in a standard procedure partially purified aldose reductase is isolated in known manner from bovine lenses. The percentage inhibition of this enzyme's ability in vitro to catalyse the reduction of aldoses to polyhydric alcohols, and particularly to reduce glucose to sorbitol, caused by a test compound can then be determined using standard spectrophotometric methods.

In typical examples of the above in vitro test, the Compound had an $IC_{50}$ of $20 \times 10^{-8}M$ and the known, structurally related, (4-aminophenylsulphonyl)nitromethane, had an $IC_{50}$ of about $100 \times 10^{-8}M$.

The Compound (or a pharmaceutically acceptable salt thereof) will primarily be administered systemically (generally by mouth) to a warm-blooded animal to produce a therapeutic or prophylactic effect mediated by inhibition of the enzyme aldose reductase, for example at a daily dose in the range of 1 to 40 mg/kg. In man it is envisaged that a total daily dose in the range, for example, 15 to 800 mg. per man will be administered, given if necessary, in divided doses. However, the precise amount of the Compound administered will naturally vary somewhat, for example, with the age and sex of the patient and the severity and extent of the condition being treated.

The Compound (or a pharmaceutically acceptable salt thereof) may also be administered topically, for example by direct topical administration to the tissue or organ in which inhibition of the enzyme is required, for example, to the eye. The precise amount of the Compound administered will necessarily depend on the formulation used. Thus, for example, when a solution is administered, a concentration of the Compound containing up to 0.01% by weight will generally be used. Similarly, when an ointment is administered a concentration of the Compound of up to 2% by weight will generally be used. Topical formulations of the Compound (or a pharmaceutically acceptable salt thereof) may be administered to the eye of an animal, for example, man or dog, requiring treatment and/or prevention of diabetic cataracts or retinopathy, in a conventional manner, for example, using a drop or eyewash topical formulation.

The Compound may be conveniently administered at or about the same time as one or more other agents which are known to have a useful effect in the treatment of diabetes or galactosemia, for example, a hypoglycaemic agent such as tolbutamide, chlorpropamide or glybenclamide. Any one or more such agents may also be conveniently present as an additional active ingredient in a composition according to the present invention.

The invention will now be illustrated by the following non-limiting Examples (of which Example 2 describes the production of an intermediate) and in which, unless otherwise stated:-

(i) solvents were removed by rotary evaporation in vacuo with a bath temperature of 40-50°C;

(ii) all operations were carried out at room temperature, that is in the range 18-26°C;

(iii) column and flash chromatography was carried out on silica (Merck Art. 7736) and medium pressure liquid chromatography (MPLC) on silica (Merck Art. 9385), both materials available from E Merck and Co., Darmstadt, West Germany;

(iv) end-products were characterised by microanalysis, NMR and/or mass spectoscopy; and

(v) yields are for illustration only and are not necessarily the maximum attainable by diligent process development.

## Example 1

(4-Acetamido-2,6-dimethylphenylsulphonyl)nitromethane (11.5g, 40mM) is added in one portion to a boiling mixture of concentrated hydrochloric acid (22 ml), water (110 ml) and ethanol (45 ml). The mixture is stirred at reflux until a clear solution formed (about 20 minutes) and then for a further 10 mins. The hot reaction mixture is then poured into an excess of ice-cold saturated sodium bicarbonate solution. The aqueous mixture is extracted with ethyl acetate. The combined extracts are washed with brine, dried ($MgSO_4$) and the solvent removed by evaporation to give **(4-amino-2,6-dimethylphenylsulphonyl)nitromethane**, as a solid, m.p. 132-133°C [after recrystallisation from ethanol] in 73% yield; NMR($d_6$-DMSO, 200MHz): 2.39(6H, s), 6.19(4H, s), 6.35(2H,s); microanalysis, found: C,44.5; H,4.9; N,11.6%; $C_9H_{12}N_2O_4S$ requires: C,44.3; H,4.9; N,11.5%.

## Example 2

Nitromethane (6.72 ml, 124 mM) is added to a stirred solution of sodium methoxide (3.01 g, 55.8 mM) in N,N-dimethylformamide (DMF; 250 ml), cooled to 0°C in an ice-bath. When the addition was complete, stirring is continued for an additional 30 minutes at 0°C. 4-Acetamido-2,6-dimethylbenzene sulphinic acid sodium salt (11.59 g, 56 mmol) is then added, followed immediately by iodine (7.2 g, 28.3 mmol). The mixture is stirred for 16 hours and allowed to attain room temperature. A concentrated solution of aqueous sodium sulphite is then added to partially decolourise the reaction mixture, which latter was is then poured into water (about 1 litre) and acidified with 2M hydrochloric acid. The aqueous mixture is extracted with ethyl acetate. The combined extracts are washed with water, then with brine, and dried ($MgSO_4$). The solvent is removed by evaporation and the residue is purified by medium pressure liquid chromatography (MPLC) on silica, eluting with ethyl acetate-hexane (1:10 v/v, gradually increasing to 1:5 v/v) to give **(4-acetamido-2,6-dimethylphenylsulphonyl)nitromethane** as a solid, m.p. 179-180°C [purified by trituration with methanol] in 21% yield; NMR ($d_6$-DMSO, 200MHz): 2.08(3H, s), 2.54(6H, s), 6.42(2H, s), 7.51(2H, s), 10.26(1H, s); microanalysis, found: C,46.2; H,5.0; N,9.7%; $C_{11}H_{14}N_2O_5S$ requires: C,46.15; ,4.9; N,9.8%.

The starting sulphinic acid may be obtained as follows:-

N-Acetyl-3,5-dimethylaniline (obtained as a solid, 138°C, by acetylation of 3,5-dimethylaniline) is reacted with an excess of chlorosulphonic acid at 60°C, using an analogous procedure to that described in Organic Syntheses, Coll. Vol.I, at page 85, to give 4-acetamido-2,6-dimethylbenzene sulphonyl chloride as a solid [thin layer chromatographic analysis (TLC): Rf ca. 0.27 (SiO2: ethyl acetate/hexane 1:1 v/v)] in about 90% yield, which is used without drying or characterisation.

The above sulphonyl chloride (10.95 g, 50 mmol) is added in portions to a vigorously stirred solution of sodium bicarbonate (8.4 g, 100 mmol) and anhydrous sodium sulphite (12 g, 95 mmol) in water (50 ml) at 70-80°C. The temperature is kept at 70-80°C by intermittent heating. When the addition is complete, the mixture is heated and stirred at 70-80°C for a further hour. The mixture is then allowed to cool to room temperature during 4 hours and acidified with 2M hydrochloric acid. The precipitated solid is collected by filtration, washed with water, air dried and to give 4-acetamido-2,6-dimethylbenzene sulphinic acid, as a solid in 56-87% yield; TLC: Rf ca. 0.02 (silica: ethyl acetate). This acid is converted to its sodium salt by addition to a solution of sodium methoxide (1 equivalent) in methanol and evaporation of the resultant solution. The sodium salt is used without purification or characterisation.

## Example 3

(4-Acetamido-2,6-dimethylphenylsulphonyl)nitromethane (1.0g) is added to 2M sodium hydroxide solution (10ml). The mixture is stirred at 80°C for 1 hour and then poured into ice-water (50ml) containing acetic acid (1.2ml). The resultant white precipitate is collected by filtration, washed with water and dried in vacuo ($P_4O_{10}$) to give **(4-amino-2,6-dimethylphenylsulphonyl)nitromethane**, as a solid, m.p. 132-133°C [after recrystallisation from ethanol] in essentially quantitative yield, essentially identical to the material obtained in Example 1.

## Example 4

The following illustrate representative pharmaceutical dosage forms containing the Compound, or a non-toxic salt thereof, for therapeutic or prophylactic use in humans:

```
(a)  Tablet I                                      mg/tablet
     Compound      ................................ 100
     Lactose Ph.Eur............................... 182.75
     Croscarmellose sodium........................  12.0
     Maize starch paste (5% w/v paste)............   2.25
     Magnesium stearate...........................   3.0
```

```
(b)  Tablet II                                     mg/tablet
     Compound      ................................  50
     Lactose Ph.Eur............................... 223.75
     Croscarmellose sodium........................   6.0
     Maize starch.................................  15.0
     Polyvinylpyrrolidone (5% w/v paste)..........   2.25
     Magnesium stearate...........................   3.0
```

```
(c)  Tablet III                                    mg/tablet
     Compound      ................................   1.0
     Lactose Ph.Eur...............................  93.25
     Croscarmellose sodium........................   4.0
     Maize starch paste (5% w/v paste)............   0.75
     Magnesium stearate...........................   1.0
```

```
(d)  Capsule                                       mg/capsule
     Compound      ................................  10
     Lactose Ph.Eur ..............................  488.5
     Magnesium stearate ..........................   1.5
```

The above formulations may be obtained by conventional procedures well known in the pharmaceutical art. The tablets (a)-(c) may conveniently be enteric coated by conventional means, for example to provide a coating of cellulose acetate phthalate.

**Claims**

1. The novel compound (4-amino-2,6-dimethylphenylsulphonyl)nitromethane, or a pharmaceutically acceptable salt thereof.

2. A salt as claimed in claim 1 which is a base-addition salt.

3. A salt as claimed in claim 2 which is selected from alkali metal, alkaline earth metal and ammonium salts, and salts with organic bases giving physiologically acceptable cations.

4. A salt as claimed in claim 1 which is selected from salts with a hydrogen halide, sulphuric acid and phosphoric acid.

5. (4-Amino-2,6-dimethylphenylsulphonyl)nitromethane, or a pharmaceutically acceptable salt thereof, for use as an inhibitor of the enzyme aldose reductase.

6. A pharmaceutical composition which comprises (4-amino-2,6-dimethylphenylsulphonyl)nitromethane, or a pharmaceutically acceptable salt thereof, together with a pharmaceutically acceptable diluent or carrier.

7. A composition as claimed in claim 6 which is in a form suitable for oral administration.

8. A process for the manufacture of the novel compound (4-amino-2,6-dimethylphenylsulphonyl)nitromethane, or a pharmaceutically acceptable salt thereof, which is characterised by:-
   a) removing a protecting group from a protected form of said compound;
   b) reacting an alkali metal salt of 4-amino-2,6-dimethylbenzene sulphinic acid with nitromethane and iodine; or
   (c) oxidising the thioether, (4-amino-2,6-dimethylphenylthio)nitromethane;
   whereafter, when a pharmaceutically acceptable salt is required, said compound is reacted with an appropriate base or acid affording a physiologically acceptable ion.

9. An N-acyl derivative of (4-amino-2,6-dimethylphenylsulphonyl)nitromethane, wherein the N-acyl is selected from N-(1-6C)alkanoyl and N-benzoyl.

10. (4-Acetamido-2,6-dimethylphenylsulphonyl)nitromethane.


**Patentansprüche**

1. Die neue Verbindung (4-Amino-2,6-dimethylphenylsulfonyl)nitromethan sowie die pharmazeutisch zulässigen Salze davon.

2. Salze nach Anspruch 1, welche Basenadditionssalze sind.

3. Salze nach Anspruch 2, welche ausgewählt sind aus Alkalimetall-, Erdalkalimetall- und Ammoniumsalzen sowie Salzen mit organischen Basen, die physiologisch zulässige Kationen liefern.

4. Salze nach Anspruch 1, welche ausgewählt sind aus Salzen mit Halogenwasserstoffen, Schwefelsäure und Phosphorsäure.

5. Die Verwendung von (4-Amino-2,6-dimethylphenylsulfonyl)nitromethan oder eines pharmazeutisch zulässigen Salzes davon als Inhibitor für das Enzym Aldosereductase.

6. Pharmazeutische Zusammensetzung, welche (4-Amino-2,6-dimethylphenylsulfonyl)nitromethan oder ein pharmazeutisch zulässiges Salz davon gemeinsam mit einem pharmazeutisch zulässigen Verdünnungs- oder Trägermittel enthält.

7. Zusammensetzung nach Anspruch 6, welche eine für orale Verabreichung geeignete Form aufweist.

8. Verfahren zur Herstellung der neuen Verbindung (4-Amimo-2,6-dimethylphenylsulfonyl)nitromethan oder eines pharmazeutisch zulässigen Salzes davon, gekennzeichnet durch

a) Entfernung einer Schutzgruppe von einer geschützten Form der genannten Verbindung;

b) Umsetzung eines Alkalimetallsalzes von (4-Amino-2,6-dimethylbenzolsulfinsäure mit Nitromethan und Jod; oder

c) Oxidation des Thioethers (4-Amino-2,6-dimethylphenylthio)nitromethan;

worauf, wenn ein pharmazeutisch zulässiges Salz gewünscht wird, die genannte Verbindung mit einer geeigneten Base oder Säure umgesetzt wird, die ein physiologisch zulässiges Ion liefert.

9. N-Acylderivate von (4-Amino-2,6-dimethylphenylsulfonyl)nitromethan, wobei das N-Acyl ausgewählt ist aus N-(1-6C)Alkanoyl und N-Benzoyl.

10. (4-Acetamido-2,6-dimethylphenylsulfonyl)nitromethan.

**Revendications**

1. A titre de composé nouveau, le (4-amino-2,6-diméthylphénylsulfonyl)-nitrométhane ou un sel acceptable du point de vue pharmaceutique de ce composé.

2. Sel suivant la revendication 1, qui est un sel d'addition de base.

3. Sel suivant la revendication 2, qui est choisi entre des sels de métaux alcalins, de métaux alcalino-terreux et d'ammonium et des sels formés avec des bases organiques donnant des cations acceptables du point de vue physiologique.

4. Sel suivant la revendication 1, qui est choisi parmi des sels formés avec un halogénure d'hydrogène, l'acide sulfurique et l'acide phosphorique.

5. Le (4-amino-2,6-diméthylphénylsulfonyl)nitrométhane ou un sel acceptable du point de vue pharmaceutique de ce composé, destiné à être utilisé comme inhibiteur de l'enzyme aldose-réductase.

6. Composition pharmaceutique, qui comprend du (4-amino-2,6-diméthylphénylsulfonyl)nitrométhane ou un sel acceptable du point de vue pharmaceutique de ce composé, en association avec un diluant ou support acceptable du point de vue pharmaceutique.

7. Composition suivant la revendication 6, qui est sous une forme qui convient pour l'administration orale.

8. Procédé de production du composé nouveau (4-amino-2,6-diméthylphénylsulfonyl)nitrométhane ou d'un sel acceptable du point de vue pharmaceutique de ce composé, qui est caractérisé par :

a) l'élimination d'un groupe protecteur d'une forme protégée dudit composé ;

b) la réaction d'un sel de métal alcalin de l'acide 4-amino-2,6-diméthylbenzène-sulfinique avec le nitrométhane et l'iode ; ou

c) l'oxydation du thioéther que constitue le (4-amino-2,6-diméthylphénylthio)nitrométhane ;

après quoi, lorsqu'on désire un sel acceptable du point de vue pharmaceutique, on fait réagir ledit composé avec une base ou un acide approprié apportant un ion acceptable du point de vue physiologique.

9. Dérivé $\underline{N}$-acylé du (4-amino-2,6-diméthylphénylsulfonyl)nitrométhane dans lequel le groupe $\underline{N}$-acyle est choisi entre des groupes $\underline{N}$-alcanoyle en $C_1$ à $C_6$ et le groupe $\underline{N}$-benzoyle.

10. Le (4-acétamido-2,6-diméthylphénylsulfonyl)nitrométhane.